## Europäisches Patentamt

## European Patent Office

## Office européen des brevets

(19)

(11) **EP 1 064 918 B1**

(12) **FASCICULE DE BREVET EUROPEEN**

(45) Date de publication et mention
de la délivrance du brevet:
**25.09.2002 Bulletin 2002/39**

(51) Int Cl.$^7$: **A61K 7/06**

(21) Numéro de dépôt: **00401593.9**

(22) Date de dépôt: **06.06.2000**

(54) **Procédé cosmétique capillaire contenant des particules métalliques pour améliorer la brillance des cheveux**

Kosmetisches Verfahren zur Haarbehandlung mit Metallpartikeln zur Verbesserung des Haarglanzes

Hair cosmetic process using metallic particles for improving hairgloss

(84) Etats contractants désignés:
**DE ES FR GB IT**

(30) Priorité: **25.06.1999 FR 9908176**

(43) Date de publication de la demande:
**03.01.2001 Bulletin 2001/01**

(73) Titulaire: **L'OREAL**
**75008 Paris (FR)**

(72) Inventeurs:
  • **Samain, Henri**
    **91570 Bièvres (FR)**
  • **Dauga, Christophe**
    **92300 Levallois-Perret (FR)**
  • **Giroud, Franck**
    **92110 Clichy (FR)**

(74) Mandataire: **Bourdeau, Françoise**
    **L'OREAL,**
    **Département Propriété Industrielle,**
    **6, rue Bertrand Sincholle**
    **92585 Clichy Cedex (FR)**

(56) Documents cités:
    **EP-A- 0 887 067          WO-A-97/38667**
    **WO-A-98/43600          DE-U- 9 210 516**
    **FR-A- 2 184 890          FR-A- 2 719 218**

    • **DATABASE WPI Week 8040 Derwent**
      **Publications Ltd., London, GB; AN 1980-70454c**
      **XP002132999 & JP 55 108812 A (UMEZAWA)**

## Description

L'invention a pour objet un procédé cosmétique capillaire procurant aux cheveux de la brillance et comprenant l'application sur les cheveux d'une composition limpide contenant des particules de type métallique en suspension dans un milieu cosmétiquement acceptable. Elle vise également l'utilisation de particules de type métallique en cosmétique capillaire dans la fabrication d'une formulation capillaire dans le but de conférer aux cheveux un effet de brillance.

Au sens de la présente invention, on entend par « composition cosmétique capillaire », des compositions de fixation et/ou de maintien des cheveux, des compositions de soin de cheveux, des compositions de conditionnement des cheveux, telles que des compositions destinées à apporter de la douceur aux cheveux, ou encore des compositions de maquillage des cheveux.

La composition cosmétique capillaire selon l'invention peut être utilisée dans une application rincée ou non rincée, et de préférence dans une application non rincée.

Il est connu des produits cosmétiques capillaires donnant aux cheveux des effets de brillance. Ces produits sont basés sur l'utilisation de molécules ou polymères qui sont soit solubilisées, soit en émulsion ou en dispersion dans un solvant cosmétique.

Toutefois, ces compositions connues ne procurent pas encore aux cheveux la brillance souhaitée. De plus, si un effet de brillance peut parfois être obtenu, ce dernier n'est pas rémanent au shampooing et il manque d'intensité.

Pour obtenir un effet de brillance, il est également connu d'utiliser des compositions riches en substances hydrophobes lubrifiantes, telles que des huiles ou cires organiques ou des silicones. Toutefois, là encore, l'effet de brillance obtenu avec ces matières lubrifiantes manque d'intensité et donne en général à la chevelure un aspect artificiel. En outre, ces substances présentent l'inconvénient d'apporter, après application à la chevelure, un toucher gras ou collant.

Le problème posé par l'invention est de trouver des compositions cosmétiques capillaires qui procurent à la chevelure une brillance intense et naturelle sans présenter les inconvénients exprimés ci-dessus.

De manière surprenante et inattendue, la Demanderesse a découvert qu'il était possible d'apporter aux cheveux une brillance intense sans altérer leur propriétés cosmétiques telle que leur douceur naturelle, en utilisant des particules métalliques dans un support cosmétique approprié, de telle sorte que la composition cosmétique capillaire soit limpide.

L'invention a pour objet un procédé cosmétique capillaire pour apporter aux cheveux de la brillance comprenant l'application sur les cheveux d'une composition cosmétique capillaire limpide comprenant des particules d'éléments métalliques, de métalloïdes, d'alliages métalliques, de carbures ou de nitrures d'éléments métalliques ou de métalloïdes, en suspension dans un milieu cosmétiquement acceptable.

En particulier, le procédé conforme à l'invention est un procédé de fixation et/ou de maintien des cheveux, un procédé de conditionnement des cheveux, ou un procédé de maquillage des cheveux.

Un autre objet de l'invention concerne l'utilisation particules d'éléments métalliques, de métalloïdes, d'alliages métalliques, de carbures ou de nitrures d'éléments métalliques ou de métalloïdes, pour la fabrication de formulation cosmétique capillaire, dans le but d'apporter aux cheveux de la brillance.

Conformément à le présente invention, on utilise le test décrit ci-après pour déterminer si une composition est « limpide ».

On prépare une composition à caractériser en diluant ou en concentrant le cas échéant la composition cosmétique capillaire, de façon à atteindre une concentration de 0,05 % en poids en particules d'éléments métalliques, de métalloïdes, d'alliages métalliques, de carbure ou de nitrure d'éléments métalliques ou de métalloïdes par rapport au poids total de la composition. On agite cette composition à caractériser pendant dix secondes, et on la transfère instantanément dans une cellule de spectromètre ultraviolet UV-2100 (plage spectrale 240-800nm) commercialisé par Shimadzu.

La cellule contenant la composition à caractériser est placée sur le chemin optique d'un faisceau lumineux. L'éclairage est un éclairage en faisceaux parallèles. On mesure la transmission optique de la composition sur le spectre du visible (400 - 700 nm). Le parcours optique du faisceau dans la cuve est de 1 cm. La taille du faisceau est de 3 mm de largeur et 5 mm de hauteur.

On détermine d'une part, la transmission directe en mesurant l'intensité lumineuse transmise dans l'axe du faisceau cylindrique. On détermine d'autre part, la transmission totale en mesurant l'intensité lumineuse transmise dans toutes les directions de l'espace au moyen d'une sphère d'intégration.

La composition à caractériser est maintenue à une température voisine de 25°C.

La composition est « limpide » au sens de la présente invention si la transmission directe est supérieure à 0,9 fois la transmission totale.

Au sens de la présente invention, on entend par "taille de particule" la dimension maximale qu'il est possible de mesurer entre deux points diamétralement opposés de la particule. La taille est déterminée en mesurant leur surface spécifique par microscopie par balayage électronique à transmission BET.

Les particules selon l'invention peuvent par exemple avoir la forme de sphères, de paillettes, ou des formes totalement aléatoires.

Avantageusement, la taille des particules est inférieure à 200 nm, et de préférence, elle est comprise entre 1 nm et 100 nm.

Avantageusement, le métal constituant les particules conformes à la présente invention est choisi parmi l'or, l'argent, l'indium, le cuivre, le silicium ou l'yttrium, l'argent étant particulièrement préféré.

Parmi les carbures et nitrures métalliques, on peut citer de façon non exhaustive: le carbure de silicium (SiC) et le nitrure de silicium ($Si_3N_4$).

Selon le métal choisi, on peut améliorer les performances des compositions en les maintenant à l'abri de l'air, afin de limiter leur oxydation.

On réalise, de préférence, des compositions dans lesquelles les particules sont présentes à une concentration en poids par rapport au poids total de la composition comprise entre 0,0001 et 10 %.

On peut également introduire dans les compositions selon l'invention des particules métalliques de nature différentes, par exemple des particules de différents éléments métalliques ou des particules d'éléments métalliques et métalloïdes.

Selon un mode préféré de l'invention, on mélange les métaux pour que la température de fusion du mélange formant les particules soit inférieure à 200°C.

Les compositions conformes à l'invention contiennent, de préférence, un solvant organique choisi dans le groupe comprenant les alcools en $C_1$ à $C_4$ tels que l'éthanol ou l'isopropanol, les alcanes en $C_5$ à $C_{10}$, l'acétone, la méthyléthylcétone, l'acétate de méthyle, l'acétate de butyle, l'acétate d'éthyle, le diméthoxyéthane, le diéthoxyéthane et leurs mélanges.

Elles peuvent contenir en outre des additifs cosmétiques usuels choisis parmi les agents adhésifs, les agents réducteurs comme les thiols, les silanes comme l'amino propyl triéthoxysilane, les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères fixants ou non, les silicones volatiles ou non, notamment les silicones anioniques, les polyols, les protéines et les vitamines.

Les compositions conformes à l'invention peuvent être conditionnées sous diverses forme, notamment dans un dispositif aérosol.

Selon un mode de mise en oeuvre préférentiel du procédé selon l'invention, on applique sur les cheveux un agent réducteur ou un agent adhésif avant d'appliquer la composition limpide comprenant les particules. En outre, on peut chauffer les cheveux avant, après ou pendant l'application de la composition limpide comprenant les particules, en particulier à une température supérieure à la température de fusion des particules métalliques.

En utilisant le procédé conforme à l'invention, il devient possible d'obtenir, sur des cheveux châtains non colorés, une brillance homogène sur toute la chevelure, une coloration gris argent très légère ou des reflets violets bleutés.

Au sens de la présente invention, la brillance est mesurée soit de manière qualitative par des test sensoriels, soit de manière quantitative à l'aide d'un photogonioréflectomètre comprenant un détecteur photomultiplicateur de référence R928S commercialisé par Hamamatsu.

Pour la mesure quantitative, on utilise une mèche plate de 20 mm de largeur, pesant 2,7 grammes et constituée de cheveux propres et secs, mesurant 27 centimètres de longs. On place cette mèche sur le support du photogonioréflectomètre et on l'éclaire au moyen d'une source lumineuse avec fibre optique, de sorte que la faisceau incident soit de 10 mm. La source lumineuse est formée par une lampe halogène de 10 W et de 12 V. On mesure la brillance sur plusieurs positions, sur environ 14 cm. Le support comporte 5 trous de 15 mm de diamètre et pour une mesure de brillance, on fait passer le faisceau incident à travers l'un de ces trous. A l'aide d'un bras de réception, on mesure la réflexion spéculaire.

On émet la lumière sur la mèche en faisant varier l'angle d'incidence « alpha » entre 0 et ±90° par rapport à la normale de la surface des cheveux. On recueille l'intensité réfléchie par cette mèche. On cherche le maximum d'intensité réfléchie dite « intensité spéculaire » et notée « R ». Généralement, elle est obtenue pour un angle alpha compris entre -20 et -30°.

La mesure est effectuée à la température ambiante.

On mesure également, pour la même mèche, la valeur de l'intensité « D » en réflexion diffuse, c'est-à-dire à un angle alpha de +15°.

Pour une mèche, la brillance « B » est obtenue à l'aide de la relation suivante :

$$B = (R-5)/(D-5)$$

La brillance est mesurée sur la même mèche avant ($B_{avant}$) et après ($B_{après}$) mise en oeuvre du procédé selon l'invention et l'augmentation de la brillance est calculée par le rapport 100 x ($B_{après}$ / $B_{avant}$).

Selon un mode de mise en oeuvre particulièrement avantageux du procédé selon l'invention, ce dernier est mis en oeuvre pour procurer une augmentation de la brillance supérieure à 30%, et de préférence supérieure à 40 %.

Le procédé conforme à l'invention peut être mis en oeuvre en utilisant des compositions de fixation et/ou de maintien des cheveux, des compositions de soin de cheveux, des compositions de conditionnement des cheveux, telles que des compositions destinées à apporter de la douceur aux cheveux, ou encore des compositions de maquillage des cheveux.

Il est maintenant possible, en utilisant le procédé conforme à l'invention, d'apporter aux cheveux une brillance nettement supérieure à la brillance naturelle et de donner ainsi à la coiffure un aspect parfois scintillant, parfois nacré ou parfois aussi métallique.

L'invention pourra être mieux comprise à l'aide des exemples non limitatifs qui suivent et qui constituent des modes de mise en oeuvre préférentiels du procédé conforme à l'invention.

Dans les exemples, les pourcentages sont exprimés en poids et m.a. signifie matière active.

Les particules d'argent utilisées dans les exemples sont commercialisées par la Société Nanophase Technologies sous l'appellation Nanocrystalline Silver Dispersion in α-Terpineol.

**EXEMPLE COMPARATIF**

On réalise 2 compositions selon la présente invention et une composition selon l'art antérieur.

**Composition 1 (invention):**

| Nanoparticules d'argent de 15 nm (70% dans terpinéol) | 0.1% (m.a.) |
|---|---|
| Acétone | 49.95% |
| Heptane | 49.95% |

Cette composition présente une couleur brun foncé et est limpide.

Elle est appliquée à raison de 1 g pour une mèche de 2.5 g de cheveux châtains européens naturels. Les cheveux sont laissés à sécher à l'air libre et on note, par rapport aux cheveux non traités, une brillance supérieure. En outre, les cheveux sont doux, et procurent un toucher agréable.

**Composition 2 (invention):**

| Nanoparticules d'argent de 15 nm (70% dans terpinéol) | 0.1% (m.a.) |
|---|---|
| Diméthyléther          qs | 100% |

Cette composition est maintenue sous pression dans un conditionnement résistant à la pression et équipée d'un système de pulvérisation aérosol type « laque ».

Elle présente une couleur brun foncé et elle est limpide.

Elle est appliquée à raison de 1 g pour une mèche de 2.5 g de cheveux châtains européens naturels, par pulvérisation en direction de la mèche. La pulvérisation dure 2 secondes et est réalisée à 20 cm de la mèche.

Les cheveux sèchent très vite et on note, par rapport aux cheveux non traités, une brillance supérieure. Les cheveux sont doux, et procurent un toucher agréable.

On mesure l'augmentation de la brillance de façon quantitative, en mettant en oeuvre le protocole opératoire défini précédemment.

Lorsque 8 g de cette composition 2 sont appliqués sur une mèche de 2,7 g de cheveux châtains, on mesure une augmentation de 47±15 % de la brillance vis-à-vis de celle mesurée sur une mèche naturelle. Dans ce cas, la densité théorique d'argent déposée sur la mèche est de 3 mg d'argent par gramme de cheveux.

## Composition 3 (art antérieur)

| | |
|---|---|
| Silicone phénylée (Dow Corning 556 fluid cosmetic) | 0.1% |
| Alcool        qs | 100% |

Les compositions 1 (invention) et 3 (art antérieur) sont appliquées sur des mèches de cheveux naturels européens châtains, à raison de 1 g pour 2,5 g de cheveux. Puis les mèches sont laissées à sécher à l'air libre.

Pour comparer les compositions selon l'art antérieur et selon l'invention, on utilise un test sensoriel. Un jury de 8 personnes notent les caractéristiques cosmétiques des mèches:

- Qualité du toucher (de 0 à 5; 0= très mauvais, 5= très bonne)
- Douceur (de 0 à 5; 0= très rêche, 5= très bonne)
- Propreté des doigts après toucher (de 0 à 5; 0= très mauvais, 5= très propre)
- Qualité ,de la brillance (de 0 à 5; 0= aspect sale, 5= aspect parfaitement naturel)

Les résultats sont rassemblés dans le tableau I ci-après:

Tableau I

| Mèches | Qualité toucher | Douceur | Propreté des doigts | Qualité de la brillance |
|---|---|---|---|---|
| Traitée par composition 1 (invention) | 4.25 | 3.5 | 5 | 4 |
| Traitée par composition 3 (art antérieur) | 1.25 | 1.5 | 1.75 | 0.75 |

On note, pour la mèche traitée par la composition 3 conforme à l'art antérieur, que les cheveux collent les uns aux autres. On peut les séparer par un coup de peigne. Cependant, l'effet de collage des cheveux les uns aux autres réapparaît très vite donnant un aspect sale aux cheveux. A l'inverse, les cheveux traités par la composition 1 conforme à l'invention n'ont aucune tendance à se coller les uns aux autres. Les cheveux traités par la composition 1 sont également doux, et présentent au toucher, du volume, ainsi qu'une brillance de bonne qualité.

## Revendications

1. Procédé cosmétique capillaire pour apporter aux cheveux de la brillance comprenant l'application sur les cheveux d'une composition cosmétique capillaire limpide comprenant des particules d'éléments métalliques, de métalloïdes, d'alliages métalliques, de carbures ou de nitrures d'éléments métalliques ou de métalloïdes, en suspension dans un milieu cosmétiquement acceptable.

2. Procédé selon la revendication 1, **caractérisé par le fait que** les particules présentent une taille inférieur à 200 nm.

3. Procédé selon la revendication 2, **caractérisé par le fait que** les particules présentent une taille comprise entre 1 nm et 100nm.

4. Procédé selon la revendication 1à 3, **caractérisé par le fait que** le métal est choisi dans le groupe comprenant l'or, l'argent, l'indium, le cuivre, le silicium ou l'yttrium.

5. Procédé selon l'une quelconque des revendications précédentes, **caractérisé par le fait que** les particules sont présentes dans la composition à une concentration relative en poids comprise entre 0,0001 et 10 %.

6. Procédé selon l'une quelconque des revendications précédentes, **caractérisé par le fait que** le métal est de l'argent.

7. Procédé selon l'une quelconque des revendications 1 à 5, **caractérisé par le fait que** la composition comprend un mélange d'au moins deux sortes de particules, la température de fusion du mélange étant inférieure à 200 °C.

**8.** Procédé selon l'une quelconque des revendications précédentes, **caractérisé par le fait que** le support cosmétique est constitué d'au moins un solvant organique choisi dans le groupe comprenant les alcools en $C_1$ à $C_4$, les alcanes en $C_5$ à $C_{10}$, l'acétone, la méthyléthylcétone, l'acétate de méthyle, l'acétate de butyle, l'acétate d'éthyle, le diméthoxyéthane, le diéthoxyéthane et leurs mélanges.

**9.** Procédé selon l'une quelconque des revendications précédentes, **caractérisé par le fait que** la composition contient des additifs cosmétiques choisis parmi les agents adhésifs, les agents réducteurs comme les thiols, les silanes comme l'amino propyl triéthoxysilane, les corps gras, les agents épaississants, les adoucissants, les agents antimousse, les agents hydratants, les agents antiperspirants, les agents alcalinisants, les colorants, les pigments, les parfums, les conservateurs, les tensioactifs, les polymères fixants ou non, les silicones volatiles ou non, notamment les silicones anioniques, les polyols, les protéines et les vitamines.

**10.** Procédé selon l'une quelconque des revendications précédentes, **caractérisé par le fait que** la composition est conditionnée dans un dispositif aérosol.

**11.** Procédé selon l'une quelconque des revendications précédentes, **caractérisé par le fait qu'**on applique sur les cheveux un agent réducteur ou un agent adhésif avant d'appliquer la composition limpide comprenant les particules.

**12.** Procédé selon l'une quelconque des revendications précédentes, **caractérisée par le fait qu'**on chauffe les cheveux avant, après ou pendant l'application de la composition limpide comprenant les particules.

**13.** Procédé selon la revendication 12, **caractérisé par le fait qu'**on chauffe les cheveux à une température supérieure à la température de fusion des particules métalliques.

**14.** Utilisation de particules d'éléments métalliques, de métalloïdes, d'alliages métalliques, de carbures ou de nitrures d'éléments métalliques ou de métalloïdes pour la fabrication d'une formulation capillaire, dans le but d'apporter aux cheveux de la brillance.

**15.** Utilisation selon la revendication 14, **caractérisée par le fait que** les particules présentent une taille inférieure à 200 nm.

**16.** Utilisation selon la revendication 14 ou 15, **caractérisée par le fait que** le métal est choisi dans le groupe comprenant l'or, l'argent, l'indium, le cuivre, le silicium ou l'yttrium.

**17.** Utilisation selon la revendication 16, **caractérisée par le fait que** le métal est de l'argent.


**Patentansprüche**

**1.** Kosmetisches Verfahren zur Haarbehandlung, um Glanz in das Haar zu bringen, das die Applikation einer klaren kosmetischen Zusammensetzung zur Haarbehandlung auf die Haare umfasst, die Partikel von metallischen Elementen, Halbmetallen, Metalllegierungen oder Carbiden oder Nitriden von metallischen Elementen oder von Halbmetallen in Suspension in einem kosmetisch akzeptablen Medium enthält.

**2.** Verfahren nach Anspruch 1, **dadurch gekennzeichnet, dass** die Größe der Partikel unter 200 nm beträgt.

**3.** Verfahren nach Anspruch 2, **dadurch gekennzeichnet, dass** die Größe der Partikel im Bereich von 1 bis 100 nm liegt.

**4.** Verfahren nach Anspruch 1 bis 3, **dadurch gekennzeichnet, dass** das Metall unter Gold, Silber, Indium, Kupfer, Silicium oder Yttrium ausgewählt ist.

**5.** Verfahren nach einem der vorhergehenden Ansprüche, **dadurch gekennzeichnet, dass** die Partikel in der Zusammensetzung in einer gewichtsbezogenen Konzentration im Bereich von 0,0001 bis 10 % vorliegen.

**6.** Verfahren nach einem der vorhergehenden Ansprüche, **dadurch gekennzeichnet, dass** es sich bei dem Metall um Silber handelt.

**7.** Verfahren nach einem der Ansprüche 1 bis 5, **dadurch gekennzeichnet, dass** die Zusammensetzung ein Gemisch von mindestens zwei Arten von Partikeln enthält, wobei der Schmelzpunkt des Gemisches unter 200 °C liegt.

**8.** Verfahren nach einem der vorhergehenden Ansprüche, **dadurch gekennzeichnet, dass** der kosmetische Träger von mindestens einem organischen Lösemittel gebildet wird, das unter Alkoholen mit 1 bis 4 Kohlenstoffatomen, Alkanen mit 5 bis 10 Kohlenstoffatomen, Aceton, Methylethylketon, Methylacetat, Butylacetat, Ethylacetat, Dimethoxyethan, Diethoxyethan und den Gemischen dieser Verbindungen ausgewählt ist.

**9.** Verfahren nach einem der vorhergehenden Ansprüche, **dadurch gekennzeichnet, dass** die Zusammensetzung kosmetische Zusatzstoffe enthält, die unter Haftmitteln, Reduktionsmitteln, wie Thiolen, Silanen, wie Aminopropyltriethoxysilan, Fettsubstanzen, Verdickungsmitteln, reizlindernden Mitteln, Schauminhibitoren, Hydratisierungsmitteln, Antiperspirantien, Mitteln zum Alkalischmachen, Farbmitteln, Pigmenten, Parfums, Konservierungsstoffen, grenzflächenaktiven Stoffen, festigenden oder nicht festigenden Polymeren, flüchtigen oder nicht flüchtigen Siliconen und insbesondere anionischen Siliconen, Polyolen, Proteinen und Vitaminen ausgewählt sind.

**10.** Verfahren nach einem der vorhergehenden Ansprüche, **dadurch gekennzeichnet, dass** die Zusammensetzung in einer Aerosolvorrichtung konfektioniert ist.

**11.** Verfahren nach einem der vorhergehenden Ansprüche, **dadurch gekennzeichnet, dass** vor der Applikation der klaren Zusammensetzung, die die Partikel enthält, ein Reduktionsmittel oder ein Haftmittel auf die Haare aufgebracht wird.

**12.** Verfahren nach einem der vorhergehenden Ansprüche, **dadurch gekennzeichnet, dass** die Haare vor, nach oder während der Applikation der klaren Zusammensetzung, die die Partikel enthält, erwärmt werden.

**13.** Verfahren nach Anspruch 12, **dadurch gekennzeichnet, dass** die Haare auf eine Temperatur erwärmt werden, die über dem Schmelzpunkt der Metallpartikel liegt.

**14.** Verwendung von Partikeln von metallischen Elementen, Halbmetallen, Metalllegierungen oder Carbiden oder Nitriden von metallischen Elementen oder von Halbmetallen zur Herstellung einer Formulierung für die Haare, mit dem Ziel, Glanz in die Haare zu bringen.

**15.** Verwendung nach Anspruch 14, **dadurch gekennzeichnet, dass** die Größe der Partikel unter 200 nm beträgt.

**16.** Verwendung nach Anspruch 14 oder 15, **dadurch gekennzeichnet, dass** das Metall unter Gold, Silber, Indium, Kupfer, Silicium oder Yttrium ausgewählt ist.

**17.** Verwendung nach Anspruch 16, **dadurch gekennzeichnet, dass** es sich bei dem Metall um Silber handelt.

**Claims**

**1.** Cosmetic hair process to give the hair sheen, comprising the application to the hair of a clear cosmetic hair composition comprising particles of metal elements, of metalloids, of metal alloys or of carbides or nitrides of metal elements or of metalloids, suspended in a cosmetically acceptable medium.

**2.** Process according to Claim 1, **characterized in that** the particles are less than 200 nm in size.

**3.** Process according to Claim 2, **characterized in that** the particles are between 1 nm and 100 nm in size.

**4.** Process according to Claims 1 to 3, **characterized in that** the metal is chosen from the group comprising gold, silver, indium, copper, silicon and yttrium.

**5.** Process according to any one of the preceding claims, **characterized in that** the particles are present in the composition at a relative concentration by weight of between 0.0001 and 10%.

**6.** Process according to any one of the preceding claims, **characterized in that** the metal is silver.

7. Process according to any one of Claims 1 to 5, **characterized in that** the composition comprises a mixture of at least two kinds of particle, the melting point of the mixture being less than 200°C.

8. Process according to any one of the preceding claims, **characterized in that** the cosmetic support consists of at least one organic solvent chosen from the group comprising $C_1$ to $C_4$ alcohols, $C_5$ to $C_{10}$ alkanes, acetone, methyl ethyl ketone, methyl acetate, butyl acetate, ethyl acetate, dimethoxyethane and diethoxyethane, and mixtures thereof.

9. Process according to any one of the preceding claims, **characterized in that** the composition contains cosmetic additives chosen from adhesives, reducing agents such as thiols, silanes such as aminopropyltriethoxysilane, fatty substances, thickeners, softeners, antifoaming agents, moisturizers, antiperspirants, basifying agents, dyes, pigments, fragrances, preserving agents, surfactants, fixing or non-fixing polymers, volatile or non-volatile silicones, in particular anionic silicones, polyols, proteins and vitamins.

10. Process according to any one of the preceding claims, **characterized in that** the composition is packaged in an aerosol device.

11. Process according to any one of the preceding claims, **characterized in that** a reducing agent or an adhesive is applied to the hair before applying the clear composition comprising the particles.

12. Process according to any one of the preceding claims, **characterized in that** the hair is heated before, during or after applying the clear composition comprising the particles.

13. Process according to Claim 12, **characterized in that** the hair is heated to a temperature above the melting point of the metal particles.

14. Use of particles of metal elements, of metalloids, of metal alloys or of carbides or nitrides of metal elements or of metalloids, for the manufacture of a hair formulation, with the aim of giving the hair sheen.

15. Use according to Claim 14, **characterized in that** the particles are less than 200 nm in size.

16. Use according to Claim 14 or 15, **characterized in that** the metal is chosen from the group comprising gold, silver, indium, copper, silicon and yttrium.

17. Use according to Claim 16, **characterized in that** the metal is silver.